(19) Europäisches Patentamt
European Patent Office
Office européen des brevets

(11) Veröffentlichungsnummer: **0 057 889**
**B1**

(12) # EUROPÄISCHE PATENTSCHRIFT

(45) Veröffentlichungstag der Patentschrift: **12.06.85**

(51) Int. Cl.⁴: **C 07 C 143/55**

(21) Anmeldenummer: **82100680.6**

(22) Anmeldetag: **01.02.82**

(54) **Verfahren zur Herstellung von 1-Alkyl-2-chlor-5-nitro-4-benzol-sulfonsäuren.**

(30) Priorität: **07.02.81 DE 3104388**

(43) Veröffentlichungstag der Anmeldung: **18.08.82 Patentblatt 82/33**

(45) Bekanntmachung des Hinweises auf die Patenterteilung: **12.06.85 Patentblatt 85/24**

(84) Benannte Vertragsstaaten: **CH DE FR GB IT LI**

(56) Entgegenhaltungen:
**DE - A - 2 240 849**
**DE - A - 2 321 332**
**DE - C - 434 402**
**US - A - 4 131 619**

(73) Patentinhaber: **BASF Aktiengesellschaft, Carl-Bosch-Strasse 38, D-6700 Ludwigshafen (DE)**

(72) Erfinder: **Tonne, Peter, Dr., Triftbrunnenweg 63, D-6730 Neustadt (DE)**
Erfinder: **Mueller, Hans-Richard, Dr., An der Tuchbleiche 9, D-6712 Bobenheim-Roxheim (DE)**
Erfinder: **Goerth, Helmut, Dr., Rubensstrasse 15, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 057 889 B1

**Beschreibung**

Die Erfindung betrifft ein Verfahren zur Herstellung von Verbindungen der Formel I

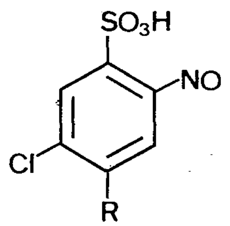

I,

in der R Alkyl ist, ausgehend von Alkylbenzolen durch aufeinanderfolgende Sulfierung, Chlorierung und Nitrierung, das dadurch gekennzeichnet ist, dass man die Alkylbenzole bei Temperaturen von 100 bis 130°C mit 2,5 bis 3 Mol konzentrierter Schwefelsäure pro Mol Alkylbenzol sulfiert, anschliessend das Reaktionsprodukt bei Temperaturen zwischen ungefähr 50 und 100°C in Abwesenheit von Katalysatoren mit ungefähr 1,1 bis 1,3 Mol Chlor umsetzt, danach bei Temperaturen zwischen 35 und 100°C mit hochkonzentrierter Salpetersäure nitriert und die Nitroverbindung isoliert.

Alklyreste R sind insbesondere solche mit 1 bis 4 C-Atomen und vorzugsweise Methyl oder Ethyl.

Das erfindungsgemässe Verfahren wird zweckmässigerweise so durchgeführt, dass man die 96 bis 98%ige Schwefelsäure zum vorgelegten Alkylbenzol so zugibt, dass die Temperatur im Bereich von 100 bis 130°C, vorzugsweise 105 bis 110°C bleibt. In der Regel ist die Sulfierung, die auch unter Überdruck ( $\leq$ 6 bar) erfolgen kann, in 1 bis 10 Stunden abgeschlossen.

Anschliessend leitet man bei Temperaturen zwischen ungefähr 50 und 100, vorzugsweise 60 bis 80°C das Chlor ein, bis die Alkylsulfonsäure verbraucht ist.

Danach wird bei Temperaturen zwischen ungefähr 35 bis 100, vorzugsweise 60 bis 70°C die hochkonzentrierte Salpetersäure in ungefähr molarer oder grösserer Menge, bezogen auf Alkylbenzol, zugegeben. Hochkonzentrierte Salpetersäure im Sinne der Erfindung ist vorzugsweise eine ungefähr 98 bis 100%ige Säure, bei geringerer Konzentration muss die Menge entsprechend erhöht werden.

Nach Abschluss der Nitrierung können die Nitroverbindungen durch Zugabe von Wasser ausgefällt und isoliert werden, zweckmässigerweise aus ungefähr 50 bis 60%iger Schwefelsäure.

Einzelheiten der Reaktionsführung können den Beispielen entnommen werden, in denen sich Angaben über Teile und Prozente, sofern nicht anders vermerkt, auf das Gewicht beziehen.

Die erfindungsgemäss hergestellten Nitroverbindungen können nach bekannten Methoden zu den Aminoverbindungen reduziert werden (z.B. durch die Béchamp-Reduktion oder durch katalytische Hydrierung). Die Aminoverbindungen sind wertvolle Diazokomponenten zur Herstellung von Pigmenten, beispielsweise seien die sogenannten Lackrot-C-Pigmente erwähnt.

Es sind schon Verfahren zur Herstellung der Verbindungen der Formel I bekannt, beispielsweise aus FIAT 1313 I, S. 123 bis 126, der DE-OS 26 27 054 oder der JA-OS 79/19 935.

Alle bekannten Verfahren liefern jedoch eine geringere Gesamtausbeute, in der Regel liegt sie um 45%.

*Beispiel 1*

93,8 Gewichtsteile Toluol werden auf 105°C erhitzt und im Laufe von 2 Stunden bei 105 bis 110°C mit 281,1 Gewichtsteilen konz. Schwefelsäure versetzt. Nach beendeter Zugabe der Schwefelsäure wird nachgerührt und dann auf 65-70°C abgekühlt. Bei dieser Temperatur leitet man im Verlauf von 12 Stunden 75 Gewichtsteile Chlor ein. Danach wird auf 60°C abgekühlt und man trägt bei 60 bis 65°C 65,3 Gewichtsteile hochkonzentrierte Salpetersäure im Laufe einer Stunde ein.

Man erhält dann 442 g eines Gemisches, das nach chromatografischer Untersuchung (HPLC-Methode) 179,5 Teile 2-Chlor-5-nitro-p-toluolsulfonsäure enthält, das sind 70% der Theorie (bezogen auf eingesetztes Toluol).

Das Gemisch wird mit Wasser so weit verdünnt, dass eine 50 gewichtsprozentige Schwefelsäure entsteht. Beim Abkühlen auf 20°C fällt die Nitroverbindung aus und wird abfiltriert.

Man erhält so 175 Gewichtsteile der Nitroverbindung mit einer Reinheit von 95% (HPLC).

Die Ausbeute beträgt 65,7% der Theorie (bezogen auf Toluol).

Durch Aufarbeitung des Filtrats kann man noch weitere 10 Teile der Nitroverbindung gewinnen.

HPLC = High Pressure Liquid Chromatography.

*Beispiel 2*

159 Teile Ethylbenzol werden auf 105 bis 108°C erhitzt und mit insgesamt 413 Teilen konz. Schwefelsäure in 2 Stunden versetzt, wobei die Temperatur nicht über 110°C steigen soll.

Nach beendeter Zugabe der Schwefelsäure wird eine Stunde nachgerührt und die Temperatur der Reaktionsmasse auf 65 bis 70°C gesenkt. Bei dieser Temperatur werden dann im Laufe von 14 Stunden 110 Teile Chlorgas eingebracht.

Man erniedrigt anschliessend die Temperatur auf 60 bis 65°C und trägt in diesem Temperaturbereich 96,5 Teile hochkonzentrierte Salpetersäure ein.

Nach beendeter Reaktion lässt man die Reaktionsmasse in eine Mischung aus 105 Teilen Wasser und 550 Teilen 60%iger Schwefelsäure einfliessen, kühlt auf 20°C ab und saugt die ausgefallene 1--Ethyl-2-chlor-5-nitrobenzolsulfonsäure-4 ab. Man erhält 303 Teile, das sind 76% der Theorie (bezogen auf Ethylbenzol).

Durch Aufarbeitung der Mutterlauge können noch weitere 16 Teile Sulfonsäure gewonnen werden, das sind 4,1% der Theorie.

Die Béchamp-Reduktion der vereinigten Na-Salzlösungen der 1-Ethyl-2-chlor-5-nitro-benzolsulfonsäure-4 liefert 261,5 Teile 5-Amino-2-chlor-p-ethylbenzolsulfonsäure, das sind 74% der theoretisch zu erwartenden Menge (bezogen auf Ethylbenzol).

Nach HPLC ist das Produkt 98,9% rein.

**Patentansprüche**

1. Verfahren zur Herstellung von 1-Alkyl-2-chlor--5-nitro-4-benzolsulfonsäuren der Formel

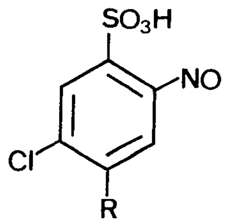

in der R Alkyl ist, ausgehend von Alkylbenzolen durch aufeinanderfolgende Sulfierung, Chlorierung und Nitrierung, dadurch gekennzeichnet, dass man die Alkylbenzole bei Temperaturen von 100 bis 130°C mit 2,5 bis 3 Mol konzentrierter Schwefelsäure pro Mol Alkylbenzol sulfiert, anschliessend das Reaktionsprodukt bei Temperaturen zwischen ungefähr 50 und 100°C in Abwesenheit von Katalysatoren mit ungefähr 1,1 bis 1,3 Mol Chlor umsetzt, danach bei Temperaturen zwischen 35 und 100°C mit hochkonzentrierter Salpetersäure nitriert und die Nitroverbindung isoliert.

2. Verfahren gemäss Anspruch 1, dadurch gekennzeichnet, dass man die Sulfierung bei Temperaturen zwischen 105 und 110°C vornimmt.

3. Verfahren gemäss Anspruch 1 und 2, dadurch gekennzeichnet, dass man pro Mol Alkylbenzol 2,65 bis 2,75 Mol 96 bis 98%ige Schwefelsäure verwendet.

4. Verfahren gemäss Anspruch 1, dadurch gekennzeichnet, dass man die Chlorierung bei Temperaturen zwischen 60 und 80°C vornimmt.

5. Verfahren gemäss Anspruch 1, dadurch gekennzeichnet, dass man die Nitrierung mit 98 bis 100%iger Salpetersäure bei Temperaturen zwischen 60 und 70°C durchgeführt.

**Claims**

1. A process for the preparation of a 1-alkyl-2--chloro-5-nitrobenzene-4-sulfonic acid of the formula

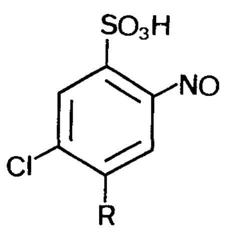

where R is alkyl, by successive sulfonation, chlorination and nitration starting from an alkylbenzene, wherein the alkylbenzene is sulfonated at from 100 to 130°C with from 2.5 to 3 moles of concentrated sulfuric acid per mole of alkylbenzene, the reaction product is then reacted at from about 50 to 100°C with from about 1.1 to 1.3 moles of chlorine in the absence of a catalyst, the product is subsequently nitrated at from 35 to 100°C with highly concentrated nitric acid, and the nitro compound is isolated.

2. A process as claimed in claim 1, wherein the sulfonation is carried out at from 105 to 110°C.

3. A process as claimed in claims 1 and 2, wherein from 2.65 to 2.75 moles of 96 to 98% strength sulfuric acid are used per mole of alkylbenzene.

4. A process as claimed in claim 1, wherein the chlorination is carried out at from 60 to 80°C.

5. A process as claimed in claim 1, wherein the nitration is carried out with 98 to 100% strength nitric acid at from 60 to 70°C.

**Revendications**

1. Procédé pour la préparation d'acides 1-alkyl--2-chloro-5-nitro-4-benzène-sulfoniques de formule

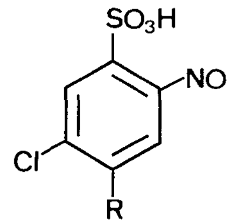

dans laquelle R est un alkyle, à partir d'alkylbenzènes par des opérations successives de sulfonation, de chloration et de nitration, caractérisé en ce qu'on sulfone les alkylbenzènes à des températures de 100 à 130°C avec 2,5 à 3 mol d'acide sulfurique concentré par mol d'alkylbenzène, puis on fait réagir le produit de la réaction, à des températures comprises entre 50 et 100°C environ et en l'absence de catalyseurs, avec 1,1 à 1,3 mol environ de chlore, on procède ensuite à une nitration avec de l'acide nitrique très concentré à une température comprise entre 35 et 100°C et on isole le composé nitré.

2. Procédé selon la revendication 1, caractérisé en ce qu'on procède à la sulfonation à des températures comprises entre 105 et 110°C.

3. Procédé selon la revendication 1 ou 2, caractérisé en ce qu'on utilise, par mol d'alkylbenzène, 2,65 à 2,75 mol d'acide sulfurique à 96 - 98%.

4. Procédé selon la revendication 1, caractérisé en ce qu'on procède à la chlorination à des températures comprises entre 60 et 80°C.

5. Procédé selon la revendication 1, caractérisé en ce qu'on effectue la nitration avec de l'acide nitrique à 98 - 100%, à des températures comprises entre 60 et 70°C.